# EUROPÄISCHE PATENTSCHRIFT

(11) **EP 0 819 116 B1**
(45) Veröffentlichungstag und Bekanntmachung des Hinweises auf die Patenterteilung: **13.06.2001**
(21) Anmeldenummer: 96907432.7
(22) Anmeldetag: 11.03.1996
(51) Int. Cl.: C07C 403/24, C07C 403/02, C09B 61/00

(54) **VERFAHREN ZUM GEWINNEN VON CAROTIN AUS PALMÖL**
PROCESS FOR OBTAINING CAROTENE FROM PALM OIL
PROCEDE D'EXTRACTION DE CAROTENE A PARTIR D'HUILE DE PALME

(30) Priorität: 20.03.1995 DE 19510098
(43) Veröffentlichungstag der Anmeldung: 21.01.1998
(73) Patentinhaber: Cognis Deutschland GmbH, 40589 Düsseldorf (DE)
(72) Erfinder: NITSCHE, Michael, D-42655 Solingen (DE); JOHANNISBAUER, Wilhelm, D-40699 Erkrath (DE); JORDAN, Volkmar, D-48565 Steinfurt (DE)
(86) Internationale Anmeldenummer: EP9601040
(87) Internationale Veröffentlichungsnummer: WO9629306

(56) Entgegenhaltungen:
- US-A- 2 460 796

## Beschreibung

Die Erfindung betrifft ein Verfahren zum Gewinnen von Carotin aus einem nativen Fett oder Öl, insbesondere aus Palmöl.

Rohes Palmöl enthält je nach Herkunft zwischen 500 und 3.000 ppm Carotinoide mit einem hohen Anteil an beta-Carotin sowie geringere Konzentrationen an Tocopherolen, Tocotrienolen und Sterolen.

Unter den Carotinoiden ist zwischen den sauerstoffhaltigen Derivaten (Xanthophyllen) und den Carotinen zu unterscheiden. Die bekanntesten Carotine sind das alpha-, beta- und gamma-Carotin sowie Lycopen. In der Natur kommt überwiegend das beta-Carotin vor, es ist das eigentliche Provitamin A. Da die Carotine natürliche Verbindungen sind und sämtlich über ausgeprägte Provitamin A-Aktivität verfügen, werden sie vielfältig in kommerziellen Anwendungen in der pharmazeutischen und in der Lebensmittelindustrie sowie bei der Kosmetikherstellung als Antioxidantien oder als Farbstoffe eingesetzt. In jüngerer Zeit wurde die tumorhemmende Wirkung von beta-Carotin mehrfach nachgewiesen, so daß es mittlerweile auch in der Krebsprophylaxe verwendet wird.

Aus dem Stand der Technik sind mehrere unterschiedliche Verfahren zum Gewinnen von Carotin aus Palmöl oder zumindest zum Aufkonzentrieren von Carotin bekannt.

In einigen bekannten Verfahren wird das Palmöl zunächst umgeestert und/oder verseift und anschließend extraktiv oder destillativ aufkonzentriert. So wird nach der US-Patentschrift 2 460 796 das Palmöl zunächst mit Methanol umgeestert. Nach dem Absetzen des Reaktionsgemisches wird die obere, aus Fettsäuremethylester mit gelöstem Carotin bestehende Phase mit einer Mischung aus Alkohol und Wasser und dann mit Wasser alleine gewaschen. Nach dem vollständigen oder teilweisen Abdestillieren der Fettsäuremethylester, die zur Seifenherstellung weiterverwendet werden können, erhält man ein Carotinkonzentrat. Bei der Destillation wird unter Vakuum bei Temperaturen bis zu 150 °C gearbeitet. Die in diesem Dokument genannte Gesamtdauer der Destillation beträgt etwa 5 Stunden.

Nachteilig in diesem bekannten Verfahren ist die thermische Beanspruchung des temperaturempfindlichen Carotins, die eine hohe Carotinausbeute verhindert.

In einem weiteren, aus der US-Patentschrift 2 572 467 bekannten Verfahren wird Palmöl zunächst verseift (Beispiele I und II). Das Reaktionsgemisch wird mit verdünnter Schwefelsäure versetzt, um die entsprechenden freien Fettsäuren zu erhalten. Nach dem Absetzen wird die Fettsäurephase in Aceton gelöst und gefiltert. Das Filtrat wird mehrfach abgekühlt und wiederum gefiltert, um einen aus Fettsäuren bestehenden Rückstand und eine carotinhaltige Lösung zu erhalten, aus der man Carotin bei -70 °C auskristallisieren läßt.

In einer Variante dieses bekannten Verfahrens tritt eine Umesterung mit Methanol an die Stelle der Verseifung des Palmöls mit anschließender Säurespaltung (Beispiele III und IV derselben US-Patentschrift).

Ein in der US-Patentschrift 2 652 433 beschriebenes Verfahren arbeitet sowohl mit einer Umesterung als auch mit einer Verseifung des Palmöls. Nach einem Neutralisieren und Filtrieren wird das rohe Palmöl mit Methanol umgeestert. Nach dem Verseifen der Esterphase schließt sich ein Extraktionsschritt mit Petrolether oder Chloroform an. Der nach dem Abdestillieren des Petrolethers erhaltene Rückstand enthält etwa 3 % Carotin.

Das 1992 veröffentlichte US-Patent 5 157 132 offenbart ein Verfahren zum Aufkonzentrieren von Carotin aus Palmöl, das ebenfalls mit einem Umesterungsschritt beginnt. Die nach dem Absetzen erhaltene esterreiche Phase wird mit Methanol und Wasser extrahiert, um eine carotinreiche Phase zu erhalten. Nach wiederholter Extraktion mit Methanol erhält man ein aufkonzentriertes Methanol-Carotin-Gemisch, aus dem der Alkohol durch Verdampfen unter Vakuum abgetrennt wird (Beispiel 1). Zusätzlich kann der carotinhaltige Fettsäuremethylester nach dem Umesterungsschritt verseift werden, bevor er mit Petrolether extrahiert wird. Hier wird also der gesamte carotinhaltige Fettsäuremethylester verseift (Beispiel II).

Nach der GB 2 218 989 A wird das rohe Palmöl nach einer Umesterung mit Methanol einer Flüssig-Chromatographie mit Methanol sowie einem Gemisch aus Hexan und Methanol oder Chloroform als Laufmittel unterzogen, um eine carotinreiche Fraktion zu erhalten.

Nachteilig an der Extraktion von Carotin aus Fettsäuremethylester ist die Verunreinigung des Fettsäuremethylesters mit dem Extraktionsmittel, die die Weiterverarbeitung des Esters durch Hydrierung zu Fettalkohol verhindert. In der Regel läßt sich der in diesem Verfahren erhaltene Ester nicht weiterverwenden und muß entsorgt werden. Den gleichen Nachteil haben die Extraktionsverfahren, die unmittelbar vom Palmöl ohne vorherige Umesterung ausgehen.

Ein Verfahren ohne diesen Nachteil wird in der US 2 432 021 beschrieben. Dort wird Carotin durch Extraktion mit verflüssigtem Propan und anschließender Rektifikation in konzentrierter Form gewonnen. In diesem Fall läßt sich das Palmöl zwar zur Herstellung von Fettalkohol weiterverwenden, aber das Verfahren ist im industriellen Maßstab nicht wirtschaftlich durchführbar.

Des weiteren sind adsorptive Verfahren zum Aufkonzentrieren von Carotin aus der GB 691 924, der GB 1 562 794 und der US 2 484 040 bekannt.

Zusammenfassend ist festzustellen, daß die bekannten Verfahren entweder großtechnisch nicht wirtschaftlich durchzuführen sind oder die Carotinausbeute infolge thermischer und/oder chemischer Zersetzung oder geringer Selektivität zu gering ist oder daß das Palmöl bzw. das Palmölderivat nach dem Durchführen des Verfahrens nicht mehr zur Herstellung von Fettalkoholen geeignet ist.

Der Erfindung liegt daher die Aufgabe zugrunde, in einem großtechnisch wirtschaftlich durchführbaren Verfahren zum Gewinnen von Carotin aus einem nativen Fett oder Öl, insbesondere aus Palmöl, eine Ausbeute von mindestens etwa 80 % zu erreichen und gleichzeitig ein für die Weiterverarbeitung zu Fettalkohol geeignetes natives Öl bzw. Ölderivat, z. B. Alkylester, zur Verfügung zu stellen. Das eingesetzte Palmöl soll durch das erfindungsgemäße Verfahren also nicht verunreinigt werden, damit es für diese Weiterverarbeitung geeignet ist.

Diese Aufgabe wird erfindungsgemäß dadurch gelöst, daß man
1. das native Fett oder Öl mit einem Alkanol der C-Kettenlänge bis 4, insbesondere mit Methanol, katalytisch zu Fettsäurealkylester und Glycerin in an sich bekannter Weise umsetzt (Umesterung),
2. die Esterphase des Reaktionsgemisches einer Destillation zum Abtrennen der Fettsäurealkylester unterzieht,
3. den in der zweiten Verfahrensstufe erhaltenen Destillationsrückstand mit einer Base, vorzugsweise Kaliumoder Natriumhydroxid umsetzt (Verseifung),
4. aus dem in der dritten Verfahrensstufe erhaltenen Produkt mit einem geeigneten Lösungsmittel gemisch Carotin extrahiert und schließlich
5. die Extraktphase durch Eindampfen bei einer Temperatur von höchstens 120°C aufkonzentriert.

Das erfindungsgemäße Verfahren ermöglicht die Herstellung eines Carotinkonzentrates mit einem Carotingehalt von 4 bis 100 Gew.-%, vorzugsweise 10 bis 90 und besonders bevorzugt 18 bis 70 Gew.-% aus einem nativen Fett oder Öl mit einer Carotinausbeute von mindestens etwa 80 % bezogen auf das eingesetzte Fett oder Öl. Gleichzeitig wird nahezu die gesamte Menge an eingesetztem Öl in Form von Fettsäurealkylester für die anschließende Hydrierung zu Fettalkohol zur Verfügung gestellt. Mit dem erfindungsgemäßen Verfahren wird damit neben dem Carotinkonzentrat und dem durch die Umesterung gewonnenen Glycerin ein weiterer Wertstoff hergestellt.

Erreicht wird dies durch die genannte destillative Abtrennung des Alkylesters von einem carotinhaltigen Rückstand und nachfolgender Verseifung sowie durch die Beschränkung des Extraktionsschritts auf den verseiften Destillationsrückstand. Trotz der Beschränkung der Extraktion auf diesen Teil des aus der Umesterung erhaltenen Reaktionsgemisches und trotz der bei der destillativen Abtrennung notwendigen hohen Temperaturen wird die genannte Ausbeute von etwa 80 % an Carotin erreicht. Überraschend ist auch die Möglichkeit, trotz des geringen Dampfdruckunterschiedes zwischen den Alkylestern und dem Carotin bis zu 99 % des Alkylesters carotinfrei abtrennen zu können. Daher ist es auch nicht notwendig, den abgetrennten Alkylester zu reinigen, bevor er zu Fettalkohol hydriert wird.

Erfindungswesentlich ist also die Kombination von Abdestillation der Esterphase und nachfolgender Verseifung des Destillationsrückstandes vor der Extraktion.

Die im nativen Fett oder Öl vorhandenen freien Fettsäuren verestert man vorzugsweise noch vor der Umesterung mit dem genannten kurzkettigen Alkanol oder verseift sie während der Umesterung, die zu diesem Zweck in Gegenwart eines ausreichend hoch dosierten alkalischen Katalysators durchgeführt wird.

Der erste erfindungsgemäße Verfahrensschritt, die Umesterung, kann batchweise oder auch kontinuierlich in Reaktoren unterschiedlicher Bauart, zum Beispiel in Rührkesselreaktoren oder Rohrreaktoren durchgeführt werden. Ein Beispiel zur Durchführung der Umesterung in einem Rohrreaktor ist in der DE 39 32 514 Al beschrieben. Als Alkohol kann Methanol, Ethanol, n- oder iso-Propanol oder -Butanol eingesetzt werden. Vorzugsweise wird Methanol verwendet.

Wird ein alkalischer Katalysator verwendet, so ist Natriumhydroxid, Kaliumhydroxid, Natriummethylat oder Kaliummethylat bevorzugt.

Da Carotin sehr temperaturempfindlich ist, sind niedrige Reaktionstemperaturen und kleine Reaktionszeiten während der Umesterung vorteilhaft. Vorgeschlagen wird eine Reaktionstemperatur von 30 bis 110°C, eine Reaktionstemperatur von 30 bis 100°C, insbesondere von 50 bis 70 °C. Die Reaktionszeit sollte bei 10 bis 180 min, insbesondere bei 30 bis 90 min liegen.

Nach Abschluß der Umesterung erhält man ein zweiphasiges Gemisch. Die Unterphase besteht aus Glycerin und enthält kein Carotin. Die Oberphase enthält im wesentlichen Fettsäurealkylester, überschüssigen Alkohol sowie das im eingesetzten nativen Öl enthaltene Carotin. Ober- und Unterphase werden durch Dekantieren getrennt.

Im zweiten erfindungsgemäßen Verfahrensschritt wird der aus der Umesterung erhaltene Fettsäurealkylester destillativ gereinigt. Dazu wird vorgeschlagen, daß man 70 bis 99,5 Gew.-%, insbesondere 95 bis 99 Gew.-% des im Umesterungsschritt erhaltenen Fettsäurealkylesters abtrennt. In diesem Schritt wird nahezu der gesamte Anteil des Fettsäurealkylesters vom carotinhaltigen Rest abgetrennt. Der in reiner Form gewonnene Alkylester steht uneingeschränkt als hochwertiges fettchemisches Ausgangsmaterial, z. B. für die Herstellung von Fettalkoholen oder von technischen Estern, zur Verfügung. Ein weiterer Vorteil liegt darin, daß das Carotin vollständig im Destillationsrückstand verbleibt und auf diese Weise erheblich aufkonzentriert wird. Ein wesentlich reduzierter Aufwand bei den nachfolgenden Schritten zum weiterem Aufkonzentrieren ist die Folge.

Die überwiegend aus Palmitin-, Stearin-, Olein- und Linolsäure bestehenden Fettsäuren des Palmöls sind sehr langkettig und erfordern bei der destillativen Abtrennung der entsprechenden Fettsäurealkylester einen niedrigen Druck, vorzugsweise im Grob- oder Feinvakuum, und eine hohe Temperatur. Trotz der hohen Temperatur tritt jedoch keine nennenswerte Zersetzung des temperaturempfindlichen Carotins auf, wenn Verdampfer mit geringer Verweilzeit eingesetzt werden. Auf diese Weise kann eine Carotinausbeute zwischen 80 und 100 % in diesem Verfahrensschritt erreicht werden.

Daher führt man die Destillation in einer vorteilhaften Ausgestaltung der Erfindung in Fallfilm-, Dünnschichtverdampfern mit rotierenden Wischern oder in Kurzwegverdampfern (Molekularverdampfern) durch. Die Verdampfung kann einstufig durchgeführt werden. Vorteilhaft ist es jedoch, wenn man die Destillation zwei- oder mehrstufig durchführt und den Druck von Stufe zu Stufe absenkt. Als Destillationstemperaturen werden 100 bis 250 °C, insbesondere 130 bis 150 °C vorgeschlagen. Der Betriebsdruck der (letzten) Verdampferstufe sollte bei 10⁰ bis 10⁻⁴ mbar, insbesondere bei 10⁻¹ bis 10⁻³ mbar liegen. Bei einstufiger Verdampfung beziehen sich diese Drücke auf diese eine Verdampferstufe. Bei mehrstufiger Verdampfung ist es vorteilhaft, wenn der Betriebsdruck der ersten Verdampferstufe bei 1 bis 50 mbar, insbesondere bei 2 bis 20 mbar liegt.

Je nach dem Destillatanteil werden hohe Konzentrationen, nämlich bis zu 20 Gew.-%, an Carotin im Destillationsrückstand erhalten.

Dieser Rückstand wird im dritten Verfahrensschritt verseift. Er wird mit einer Lauge, vorzugsweise Kali- oder Natronlauge, bei Normaldruck und Temperaturen zwischen 80 °C und 120 °C umgesetzt. Vorzugsweise gibt man Wasser bis zur fünffachen Gewichtsmenge des Destillationsrückstandes dem Reaktionsgemisch unmittelbar oder während der Verseifung zu, um das Löslichkeitsverhalten im nachfolgenden Extraktionsschritt zu verbessern.

Der vierte und vorletzte Verfahrensschritt besteht aus der Extraktion des Carotins.

Zur Extraktion wird der verseifte Destillationsrückstand intensiv mit einem Lösungsmittelgemisch vermischt, das vorzugsweise aus einer polaren und einer unpolaren Komponente besteht. Bei der Auswahl des Lösungsmittelgemisches sollte darauf geachtet werden, daß einerseits keine vollständige Löslichkeit des verseiften Rückstandes bzw. der wäßrigen Seifenlösung, aber andererseits eine sehr gute Löslichkeit des im verseiften Rückstand enthaltenen Carotins vorliegt. Als unpolares Lösungsmittel werden geradkettige oder verzweigte Kohlenwasserstoffe mit 4 bis 12 Kohlenstoffatomen und als polare Lösungsmittelkomponente Aceton oder Tetrahydrofuran vorgeschlagen. Andere Lösungsmittel sind jedoch auch möglich.

Überraschenderweise zeigte es sich, daß das Carotin in nur einem einzigen Extraktionsschritt vollständig aus dem verseiften Destillationsrückstand extrahiert werden kann. Nach der Extraktion erhält man eine gelb gefärbte Unterphase, die Seife und Wasser enthält, sowie eine orangerot gefärbte Oberphase (Extraktphase), die aus den Lösungsmitteln und Carotin besteht.

Als letzter Verfahrensschritt wird die Extraktphase eingedampft. Zunächst dampft man das Lösungsmittel bei Normaldruck ab. Nachdem etwa 80 bis 95 % der Lösungsmittelmenge abgetrennt worden sind, wird das Verfahren vorzugsweise im Vakuum fortgesetzt, um eine möglichst vollständige Abtrennung der Lösungsmittel zu erreichen.

Die Temperatur des Rückstandes sollte während der Eindampfung möglichst niedrig gehalten werden. Vorgeschlagen wird eine Temperatur von höchstens 120 °C beim Eindampfen der Extraktphase, die auch am Ende der Eindampfung nicht überschritten werden sollte.

Das abgedampfte Lösungsmittel ist carotinfrei und kann erneut zur Extraktion eingesetzt werden. Der Rückstand aus der Eindampfung enthält zwischen 20 und 95 Gew.-% Carotin sowie Spuren von Tocopherolen, Tocotrienolen und Sterolen.

Das nach dem erfindungsgemäßen Verfahren erhaltene Carotin eignet sich für den Einsatz in Lebensmitteln, Kosmetika sowie Pharmazeutika und zwar als Farbstoff und Konservierungsmittel. Eine weitere Anwendung liegt in der Krebspropylaxe. Zur Konfe ktionierung kann das aus dem Eindampfungsschritt erhaltene Carotin mit geeigneten Ölen, z. B. Sonnenblumenöl, auf eine Konzentration zwischen 1 und 50 Gew.-% verdünnt werden.

Das nachfolgende Beispiel erläutert den Gegenstand der Erfindung näher, ohne ihn darauf einzuschränken.

### Beispiel

In einem Rührkessel mit 5 1 Volumen wurden 3.000 g rohes malaysisches Palmöl mit einem Carotingehalt von 900 ppm und einer Säurezahl von 9,3 mit 1.200 g Methanol umgesetzt. Zur Verseifung der freien Fettsäuren und als Katalysator wurden 36,5 g Natriummethylat als 30 %ige methanolische Lösung zugesetzt. Die Reaktion wurde unter Rühren bei 60 °C und Normaldruck durchgeführt. Nach einer Stunde Reaktionszeit wurde der Rührer abgeschaltet, so daß die Trennung von Methylester- und Glycerinphase begann. Nach zwei Stunden Absetzzeit wurde die Glycerinphase abdekantiert. Die erzeugte Methylesterphase hatte eine Masse von 2.719 g und einen Gehalt von 1.000 ppm Carotin.

Der Methylester wurde anschließend destillativ in zwei Stufen abgetrennt. Dabei kam in der ersten Stufe ein Rotationsverdampfer und in der zweiten Stufe ein Kurzwegverdampfer zum Einsatz. Der erste Destillationsschritt wurde bei Normaldruck und 100 °C begonnen. Während der Destillation wurde zunächst der Druck auf 3 mbar abgesenkt und anschließend die Temperatur auf 140 °C erhöht. Die Destillatmenge betrug 2.403 g; das Destillat war carotinfrei und wasserhell. Die Rückstandsmenge betrug 304 g. Dieser Rückstand wurde in einem Kurzwegverdampfer bei 1,5 ^{*} 10⁻² mbar und 160 °C weiter aufkonzentriert, wobei als Destillat 226 g gelblich gefärbter Methylester und als Rückstand 70,3 g mit einem Carotingehalt von 3,6 % erhalten wurden.

Dieser Rückstand wurde mit 210 g Wasser und 17 g 50 %iger Natronlauge versetzt und anschließend 4 Stunden bei Normaldruck und 105 °C unter Rückfluß gekocht. Dabei wurden 295 g Seifenlösung erzeugt.

Diese Seifenlösung wurde mit 590 g eines Lösungsmittelgemisches aus 1 Teil n-Hexan und 2 Teilen Aceton versetzt und 30 min. gerührt. Nach anschließender zweistündiger Absetzzeit wurde 542 g braun gefärbte Unterphase abdekantiert. Die erhaltene Oberphase hatte eine Masse von 341 g und einen Gehalt an 0,75 % Carotin. Diese Oberphase wurde anschließend eingedampft, wobei zunächst bei Normaldruck und einer Heizmitteltemperatur von 80 °C gearbeitet wurde. Im weiteren Verlauf der Eindampfung wurde der Druck auf 30 mbar abgesenkt und die Heizmitteltemperatur auf 120 °C gesteigert. Dabei wurden 4,5 g dunkelroter Feststoff mit einem Carotingehalt von 54,1 % erhalten. Die Carotinausbeute des gesamten Prozesses beträgt somit 90 %. Das hergestellte Material kann ohne weitere Behandlung für einen Einsatz in Lebensmitteln, Kosmetika oder Pharmazeutika konfektioniert werden.

## Patentansprüche

1. Verfahren zum Gewinnen von Carotin aus einem nativen Fett oder Öl, insbesondere aus Palmöl, indem man
1. das native Fett oder Öl mit einem Alkanol der C-Kettenlänge bis 4, insbesondere mit Methanol, katalytisch zu Fettsäurealkylester und Glycerin in an sich bekannter Weise umsetzt (Umesterung),
2. die Esterphase des Reaktionsgemisches einer Destillation zum Abtrennen der Fettsäurealkylester unterzieht,
3. den in der zweiten Verfahrensstufe erhaltenen Destillationsrückstand mit einer Base, vorzugsweise Kalium- oder Natriumhydroxid umsetzt (Verseifung),
4. aus dem in der dritten Verfahrensstufe erhaltenen Produkt mit einem geeigneten Lösungsmittel gemisch Carotin extrahiert und schließlich
5. die Extraktphase durch Eindampfen bei einer Temperatur von höchstens 120°C aufkonzentriert.

2. Verfahren nach Anspruch 1,
**dadurch gekennzeichnet,**
daß man die im nativen Fett oder Öl vorhandenen freien Fettsäuren noch vor der Umesterung mit dem genannten kurzkettigen Alkanol verestert oder sie während der Umesterung verseift, die in Gegenwart eines ausreichend hoch dosierten alkalischen Katalysators durchgeführt wird.

3. Verfahren nach einem der vorhergehenden Ansprüche,
**dadurch gekennzeichnet,**
daß man die Umesterung in Gegenwart eines homogenen alkalischen Katalysators, insbesondere Natriumhydroxid, Kaliumhydroxid, Natriummethylat oder Kaliummethylat, durchführt.

4. Verfahren nach einem der vorhergehenden Ansprüche,
**gekennzeichnet durch**
eine Reaktionstemperatur von 30 bis 110 °C, insbesondere von 50 bis 70 °C, bei der Umesterung.

5. Verfahren nach einem der vorhergehenden Ansprüche, gekennzeichnet durch eine Reaktionstemperatur von 30 bis 100°C, insbesondere von 50 bis 70°C, bei der Umesterung.

6. Verfahren nach einem der vorhergehenden Ansprüche,
**gekennzeichnet durch**
eine Reaktionszeit von 10 bis 180 min, insbesondere von 30 bis 90 min, bei der Umesterung.

7. Verfahren nach einem der vorhergehenden Ansprüche,
**dadurch gekennzeichnet,**
daß man 70 bis 99,5 Gew.-%, insbesondere 95 bis 99 Gew.-% des im Umesterungsschritt erhaltenen Fettsäurealkylesters bei der Destillation abtrennt.

8. Verfahren nach einem der vorhergehenden Ansprüche,
**dadurch gekennzeichnet,**
daß man die Destillation in Fallfilm-, Dünnschichtverdampfem mit rotierenden Wischern oder in Kurzwegverdampfern (Molekularverdampfern) durchführt.

9. Verfahren nach einem der vorhergehenden Ansprüche,
**dadurch gekennzeichnet,**
daß man die Destillation zwei- oder mehrstufig durchführt und den Druck von Stufe zu Stufe absenkt.

10. Verfahren nach einem der vorhergehenden Ansprüche,
**gekennzeichnet durch**
Destillationstemperaturen von 100 bis 250 °C insbesondere von 130 bis 150 °C.

11. Verfahren nach einem der vorhergehenden Ansprüche,
**gekennzeichnet durch**
einen Betriebsdruck der (letzten) Verdampferstufe von 10⁰ bis 10⁻⁴ mbar, insbesondere von 10⁻¹ bis 10⁻³ mbar.

12. Verfahren nach einem der Ansprüche 9 bis 11,
**gekennzeichnet durch**
einen Betriebsdruck der ersten Verdampferstufe von 1 bis 50 mbar, insbesondere von 2 bis 20 mbar.

13. Verfahren nach einem der vorhergehenden Ansprüche,
**gekennzeichnet durch**
Reaktionstemperaturen von 80 °C bis 120 °C im dritten Verfahrensschritt (Verseifung).

14. Verfahren nach einem der vorhergehenden Ansprüche,
**dadurch gekennzeichnet,**
daß man Wasser bis zur fünffachen Gewichtsmenge des Destillationsrückstandes dem Reaktionsgemiach unmittelbar oder während der Verseifung zugibt.

## Claims

1. A process for recovering carotene from a native fat or oil, more particularly palm oil, characterized in that
1. the native fat or oil is catalytically reacted (transesterified) in known manner with an alkanol containing up to 4 carbon atoms, more particularly methanol, to form fatty acid alkyl ester and glycerol,
2. the ester phase of the reaction mixture is subjected to distillation to remove the fatty acid alkyl ester,
3. the distillation residue obtained in the second stage of the process is reacted with a base, preferably potassium or sodium hydroxide (saponification),
4. carotene is extracted with a suitable solvent mixture from the product obtained in the third stage of the process and, finally,
5. the extract phase is concentrated by evaporation at a temperature of at most 120°C.

2. A process as claimed in claim 1, characterized in that the free fatty acids present in the native fat or oil are esterified before transesterification with the short-chain alkanol mentioned or are saponified during the transesterification step in the presence of a sufficiently large amount of an alkaline catalyst.

3. A process as claimed in any of the preceding claims, characterized in that the transesterification step is carried out in the presence of a homogeneous alkaline catalyst, more particularly sodium hydroxide, potassium hydroxide, sodium methylate or potassium methylate.

4. A process as claimed in any of the preceding claims, characterized by a transesterification temperature in the range from 30 to 110EC and, more particularly, in the range from 50 to 70EC.

5. A process as claimed in any of the preceding claims, characterized by a transesterification temperature in the range from 30 to 100EC and, more particularly, in the range from 50 to 70EC.

6. A process as claimed in any of the preceding claims, characterized by a transesterification time of 10 to 180 minutes and, more particularly, 30 to 90 minutes.

7. A process as claimed in any of the preceding claims, characterized in that 70 to 99.5% by weight and, more particularly, 95 to 99% by weight of the fatty alkyl ester obtained in the transesterification step are removed during the distillation step.

8. A process as claimed in any of the preceding claims, characterized in that the distillation step is carried out in falling-film evaporators, in thin-layer evaporators with rotating wipers or in short-path evaporators (molecular evaporators).

9. A process as claimed in any of the preceding claims, characterized in that the distillation step is carried out in two or more stages and the pressure is reduced from stage to stage.

10. A process as claimed in any of the preceding claims, characterized by distillation temperatures in the range from 100 to 250EC and, more particularly, in the range from 130 to 150EC.

11. A process as claimed in any of the preceding claims, characterized by an operating pressure of the (last) evaporator stage in the range from 10⁰ to 10⁻⁴ mbar and, more particularly, in the range from 10⁻¹ to 10⁻³ mbar.

12. A process as claimed in any of claims 9 to 11, characterized by an operating pressure of the first evaporator stage of 1 to 50 mbar and, more particularly, 2 to 20 mbar.

13. A process as claimed in any of the preceding claims, characterized by reaction temperatures of 80EC to 120EC in the third step of the process (saponification).

14. A process as claimed in any of the preceding claims, characterized in that water in five times the quantity by weight of the distillation residue is added to the reaction mixture either directly or during the saponification step.

## Revendications

1. Procédé de production de carotène à partir d'une matière grasse ou d'une huile naturelle, en particulier de l'huile de palme, dans lequel,
1. on fait réagir la matière grasse ou l'huile naturelle avec un alkanol de longueur de chaîne allant jusqu'à 4, en particulier avec le méthanol, par voie catalytique d'une manière connue en soi, en ester d'alkyle d'acide gras et en glycérol (transestérification),
2. on soumet la phase ester du mélange réactionnel à une distillation pour séparer l'ester d'alkyle d'acide gras,
3. on fait réagir le résidu de distillation obtenu à la deuxième étape de procédé avec une base, de préférence l'hydroxyde de potassium ou de sodium, (saponification),
4. on extrait du produit obtenu à la troisième étape du procédé, le carotène avec un mélange de solvants approprié et finalement,
5. on concentre la phase d'extrait par évaporation à une température d'au maximum 120°C.

2. Procédé selon la revendication 1,
caractérisé en ce qu'
on estérifie les acides gras libres présents dans la matière grasse ou l'huile naturelle encore avant la transestérification avec l'alkanol à chaîne courte mentionné ou bien on les saponifie pendant la transestérification qui est effectuée en présence d'un catalyseur alcalin suffisamment hautement dosé.

3. Procédé selon l'une quelconque des revendications précédentes,
caractérisé en ce qu'
on effectue la transestérification en présence d'un catalyseur alcalin homogène, en particulier l'hydroxyde de sodium, l'hydroxyde de potassium, le méthylate de sodium ou le méthylate de potassium.

4. Procédé selon l'une quelconque des revendications précédentes,
caractérisé par
une température de réaction allant de 30 à 110°C, en particulier de 50 à 70°C, lors de la transestérification.

5. Procédé selon l'une quelconque des revendications précédentes,
caractérisé par
une température de réaction allant de 30 à 100°C, en particulier de 50 à 70°C, lors de la transestérification.

6. Procédé selon l'une quelconque des revendications précédentes,
caractérisé par
une durée de réaction allant de 10 à 180 min., en particulier de 30 à 90 min., lors de la transestérification.

7. Procédé selon l'une quelconque des revendications précédentes,
caractérisé ce qu'
on sépare lors de la distillation de 70 à 99,5 % en poids, en particulier de 95 à 99 % en poids, de l'ester d'alkyle d'acide gras obtenu dans l'étape de transestérification.

8. Procédé selon l'une quelconque des revendications précédentes,
caractérisé ce qu'
on effectue la distillation dans des évaporateurs à film tombant ou dans des évaporateurs en couche mince avec des balayeurs tournants ou dans des évaporateurs flash (évaporateurs moléculaires).

9. Procédé selon l'une quelconque des revendications précédentes,
caractérisé ce qu'
on effectue la distillation en deux étapes ou en plusieurs étapes et on abaisse la pression d'étape en étape.

10. Procédé selon l'une quelconque des revendications précédentes,
caractérisé par
des températures de distillation allant de 100 à 250°C, en particulier de 130 à 150°C.

11. Procédé selon l'une quelconque des revendications précédentes,
caractérisé par
une pression de service de 1a (dernière) étape d'évaporation allant de 10 à 10⁻⁴ mbars, en particulier de 10⁻¹ à 10^{-3 mbars}.

12. Procédé selon l'une quelconque des revendications 9 à 11,
caractérisé par
une pression de service de la première étape d'évaporation allant de 1 à 50 mbars, en particulier de 2 à 20 mbars.

13. Procédé selon l'une quelconque des revendications précédentes,
caractérisé par
des températures de réaction allant de 80 à 120°C à la troisième étape de procédé (saponification).

14. Procédé selon l'une quelconque des revendications précédentes,
caractérisé en ce qu'
on ajoute de l'eau en quantité en poids allant jusqu'au quintuple de la quantité en poids du résidu de distillation, au mélange réactionnel immédiatement ou pendant la saponification.
